Europäisches Patentamt

European Patent Office

Office européen des brevets

(19)

(11) Publication number: 0 529 985 A1

# EUROPEAN PATENT APPLICATION

(12)

(21) Application number: 92307690.5

(22) Date of filing: 21.08.92

(51) Int. Cl.5: C12Q 1/68, C07H 21/04, C12P 19/34

(30) Priority: 23.08.91 GB 9118262
09.07.92 GB 9214601

(43) Date of publication of application:
03.03.93 Bulletin 93/09

(84) Designated Contracting States:
AT BE CH DE DK ES FR GB GR IE IT LI LU MC
NL PT SE

(71) Applicant: Chang Pinares, Hernan Robert
40, chemin Frank-Thomas
CH-1208 Genève(CH)
Applicant: Boettger, Erik C.
1, Oskar-Winter-Strasse
W-3000 Hannover 1(DE)
Applicant: Hirschel, Bernhard J.
15, chemin Chamuses
CH-1234 Vessy(CH)

(72) Inventor: Chang Pinares, Hernan Robert
40, chemin Frank-Thomas
CH-1208 Genève(CH)
Inventor: Boettger, Erik C.
1, Oskar-Winter-Strasse
W-3000 Hannover 1(DE)
Inventor: Hirschel, Bernhard J.
15, chemin Chamuses
CH-1234 Vessy(CH)

(74) Representative: Ruffles, Graham Keith
MARKS & CLERK 57-60 Lincoln's Inn Fields
London WC2A 3LS (GB)

(54) Molecular detection and identification of a new mycobacterium causing infection in aids patients.

(57) A new mycobacterial pathogen, Mycobacterium genavense sp. nov., sequences for the 16S ribosomal RNA of the micro-organism and 16S ribosomal RNA gene sequence thereof, probes and primers specific for, capable of hybridising to, and useful in the amplification of, the 16S rRNA sequence of the micro-organism and the use of said probes and primers in the detection of M. genavense infections.

EP 0 529 985 A1

FIELD OF THE INVENTION

The present invention relates to the 16S rRNA gene (rDNA) sequence, signature sequence data for the 16S rRNA molecule, primers, oligonucleotide probes and methods useful for reliable detection and identification of a new, previously unreported, species of mycobacterium.

BACKGROUND OF THE INVENTION

The genus Mycobacterium consists of a diverse group of acid-fast bacilli which includes several important human and animal pathogens, such as Mycobacterium tuberculosis and Mycobacterium leprae. An increasing number of mycobacterial species have recently appeared as opportunistic pathogens as a result of immunosuppression induced either iatrogenically or due to disease, particularly in those patients suffering from the acquired immunodeficiency syndrome (AIDS). Moreover, it has been suggested that most of the mycobacterial species described so far are potential pathogens. Therefore, identification of mycobacterial pathogens is of great clinical and epidemiological importance.

In clinical practice two approaches are commonly used for the diagnosis of mycobacterial infections. These consist of direct identification of acid-fast bacilli in clinical samples, and identification of the mycobacterial species when cultures in solid media are positive. The first approach uses light-microscopy techniques and is rapid, but lacks sensitivity, and does not allow identification of the mycobacterial species. The second approach relies on phenotypic, biochemical and serological tests on cultures of the mycobacterial species. These methods are time-consuming and require complex and specialized tests. Thus, the reliable and rapid identification of mycobacteria remains problematical. Furthermore, the second approach is only viable if the micro-organism can be cultured on a solid medium.

The use of DNA- and RNA-based hybridization probes and the use of high performance liquid chromatography of mycobacterial mycolic acids have been proposed for identification of mycobacteria, but both of these assays require large numbers of bacteria (i.e. pure cultures) and considerable expertise, thereby preventing their widespread use with clinical samples.

An alternative approach is the use of a rapid and reliable procedure that could detect all Mycobacterium species followed by a second assay that allows species determination based on species-specific nucleotide sequences. Small subunit ribosomal RNAs (rRNAs) are suitable for this goal, because they are an essential constituent of bacterial and eukaryotic ribosomes and because the 16S rRNA sequence is functionally constrained such that is highly conserved with only rare sequence changes in certain positions. Nevertheless, the location of these changes appear as specific for a given group or species in which they take place. Furthermore, this specificity of the 16S rRNA sequence, and the information content of the molecule, is sufficient to allow both statistically valid phylogenetic analysis and species identification, once the sequence has been determined. rRNA also is present in large copy numbers, i.e. 1000 to 10,000 molecules per cell, thus enabling the development of sensitive detection assays.

A method for the determination of bacterial, and in particular mycobacterial, 16S rRNA gene sequences by direct sequencing of DNA amplified by the polymerase chain reaction (PCR) is given in FEMS Microbiol. Lett. 65:171-176, 1989; Nucleic Acids Research 17:7843-7853, 1989; Int. J. Sys. Bacteriol. 40:323-330, 1990. It is preferred that the amplified DNA is sequenced directly, i.e. without cloning procedures, because sequencing of cloned amplified DNA, due to nucleotide misincorporations, which is inherent to any DNA polymerase, can lead to false nucleotide determinations as a result of errors in the PCR. These artifacts can be overcome by direct sequencing of the amplified DNA fragment, since all random misincorporations of the polymerase are averaged out. Furthermore, DNA sequencing is preferred over RNA sequencing, because direct RNA sequencing using "universal primers" and reverse transcriptase enzyme frequently results in sequencing "anomalies". It also requires considerable amounts of rRNA and thus of bacteria. Because bacteria are known to be particularly rich in ribonuclease, obtention of intact RNA is difficult, in contrast with DNA which is easier to obtain and much more stable (FEMS Microbiol. Lett. 65:171-176, 1989; Nucleic Acids Research 17:7843-7853, 1989; Int. J. Sys. Bacteriol. 40:323-330, 1990).

The sensitive and rapid detection and differentiation of mycobacteria combining 16S rRNA gene amplification with the use of hybridization with 16S rRNA-derived nucleic acid (oligonucleotide) probes has been described in J. Clin. Microbiol. 28:1751-1759, 1990, the disclosure of which is incorporated herein by reference. Small but consistent differences in certain regions of the 16S rRNA sequence are employed as signature sequences to allow the definition of highly specific nucleic acid probes at the genus, group and species level. Morever, this approach allows detection of fewer than 10 mycobacterial cells when using M. tuberculosis as a model system (J. Clin. Microbiol. 28:1751-1759, 1990), making it suitable for direct mycobacterial speciation from clinical samples.

EP 0 529 985 A1

The principal object of the present invention is to provide means for reliable and rapid detection and identification of a new mycobacterial pathogen. Provision of the 16S rRNA gene (rDNA) sequence, signature sequence data for the 16S rRNA sequence, and nucleic acid primers and probes for the mycobacterium are related objects. The preferred probes/primers are designed on the basis of ribosomal ribonucleic acid sequences unique to the mycobacterium, and as such can detect the rRNA, rDNA or a polymerase chain reaction product of this gene. Methods for detecting the mycobacterium in human samples are also provided.

SUMMARY OF THE INVENTION

The present invention provides the 16S rRNA sequence for a new, as yet uncultured, species of mycobacterium tentatively named "Mycobacterium genavense" sp. nov.. From our studies, this mycobacterium appears to occur as an opportunistic infection in highly immunosuppressed patients suffering from the acquired immunodeficiency syndrome (AIDS).

The present invention further extends to signature sequences in the variable regions of the 16S rRNA gene sequence of the mycobacteria, primers and probes designed on the basis of signature sequences in the mycobacterial 16S rRNA sequence and methods useful for rapid and reliable detection and identification of M. genavense.

BRIEF DESCRIPTION OF THE DRAWINGS

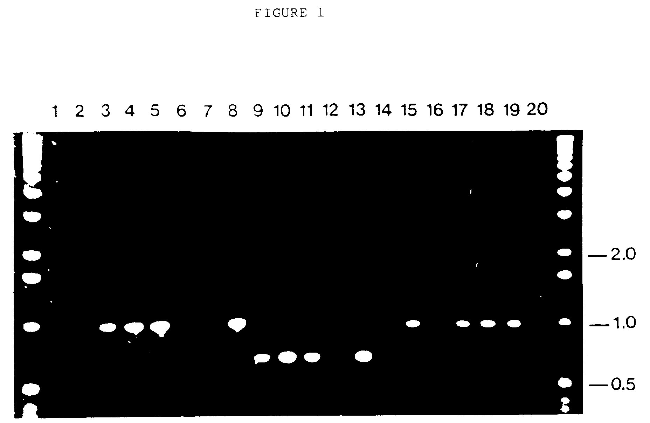

Figure 1: depicts an agarose gel electrophoresis of amplified DNA.

DETAILED DESCRIPTION OF THE INVENTION

The invention provides the 16S rRNA gene sequence of the species Mycobacterium genavense sp. nov.:

3

```
5'-GTT TGA TCC TGG CTC AGG ACG AAC GCT GGC GGC GTG CTT AAC
   ACA TGC AAG TCG AAC GGA AAG GCC TCT TCG GAG GTA CTC GAG
   TGG CGA ACG GGT GAG TAA CAC GTG GGT AAT CTG CCC TGC ACT
   TCG GGA TAA GCC TGG GAA ACT GGG TCT AAT ACC GGA TAT GAC
   CAC GGA ACG CAT GTT TTG TGG TGG AAA GCT TTT GCG GTG TGG
   GAT GGG CCC GCG GCC TAT CAG CTT GTT GGT GGG GTG ACG GCC
   TAC CAA GGC GAC GAC GGG TAG CCG CCT GAG AGG GTC CGG
   CCA CAC TGG GAC TGA GAT ACG GCC CAG ACT NCT ACG GGA GGC
   AGC AGT GGG GAA TAT TGC ACA ATG GGC GCA AGC CTG ATG CAG
   CGA CGC CGC GTG GGG GAT GAC GGC CTT CGG GTT GTA AAC CTC
   TTT CAG CAG GGA CGA AGC GCA AGT GAC GGT ACC TGC AGA AGA
   AGC ACC GGC CAA CTA CGT GCC AGC AGC CGC GGT AAT ACG TAG
   GGT GCG AGC GTT GTC NGG AAT TAC TGG CGT AAA GAG CTC GTA
   AGG TGG TTT GTC GCG TTG TTC GTG AAA ACC GGG GGC TTA ACC
   CTC GGC GTG CGG GCG ATA CGG GCA GAC TGG AGT ACT GCA GGG
   GAG ACT GGA ATT CCT GGT GTA GCG GTG AAA TGC GCA GAT ATC
   AGG AGG AAC ACC GGT GGC GAA GGC GGG TCT CTG GGC AGT AAC
   TGA CGC TGA GGA GCG AAA GCG TGG GGA GCG AAC AGG ATT AGA
   TAC CCT GGT AGT CCA CGC NGT AAA CGG TGG GTA CTA GGT GTG
   GGT TTC CTT CCT TGG AAT CCG TGC CGT AGC TAA CGC ATT AAG
   TAC CCC GCC TGG GGA GTA CGG CCG CAA GGC TAA AAC TCA AAG
   GAA TTG ACG GGG GNC CGC ACA AGC GGC GGA GCA TGT GGA TTA
   ATT CGA TGC GAC GCG AAG AAC CTT ACC TGG GTT TGA CAT GCA
   CAG GAC GCC GGC AGA GAT GTC GGT TCC CTT GTG GCC TGT GTG
   CAG GTG GTG CAT GGC TGT CGT CAG CTC GTG TCG TGA GAT GTT
   GGG TTA AGT CCC GCA ACG AGC GCA ACC CTT GTC TCA TGT TGC
```

```
CAG CGG TAA ATG CCG GGG ACT CGT GAG AGA CTG CCG GGG TCA
ACT CGG AGG AAG GTG AGG ATG ACG TCA AGT CAT CAT GCC CCT
TAT GTC AGG GCT TCA CAT GCT ACA ATG GCC AGT ACA AAG
GGC TGC GAT GCC GTA AGG TTA GCG AAT CCT TTT AAA GCC GG
TCT CAG TTC GGA TCG GGT CTG CAA CTC GAC CCC GTG AAG TC
GGA GTC GCT AGT AAT CGC AGA TCA GCA ACG CTG CGG TGA ATA
CGT TCC CGG GCC TTG TAC ACA CCG CCC GTC ACG TCA TGA AAG
TCG GTA ACA CCC GAA GCC AGT GGC CTA ACC TTT TGG AGG GAG
CTG TCG AAG GTG GGA TCG GCG-3'
```

(deposited in the EMBL data base, accesion number X60070).

In the 16S rRNA sequence N indicates a base not yet determined, which base could be adenine (A), guanine (G), cytosine (C) or thymine (T).

In the more preferred embodiments of the present invention, nucleic acid probes and primers comprise those capable of hybridizing to, or amplifying, all or a part of at least one nucleotide sequence of the 16S rRNA gene sequence of Mycobacterium genavense sp. nov..

Probes and primers according to the present invention are preferably of at least 10 nucleotides in length and are capable of hybridising to or amplifying the 16S rRNA sequence of M. genavense.

In particular, the nucleic acid probes and primers comprise those capable of hybridizing to, or amplifying, all or a part of at least one discrete nucleotide sequence selected from characteristic nucleotides within the variable species-specific regions of mycobacteria. More particularly, the nucleic acid probes and primers comprise those (sense or anti-sense) capable of hybridizing to, or amplifying, all or a part of at least one nucleotide sequence selected from the following regions, or their complementary sequences, and in positions:

157-205 (in M. genavense 16S rRNA sequence; corresponding approximately to positions 175-232 in E. coli 16S rRNA sequence):

```
5'-ACC GGA TAT GAC CAC GGA ACG CAT GTT TTG TGG TGG AAA GCT
TTT GCG G-3';
```

414-463 (in M. genavense 16S rRNA sequence; corresponding approximately to positions 440-500 in E. coli 16S rRNA sequence):

```
5'-AAA CCT CTT TCA GCA GGG ACG AAG CGC AAG TGA CGG TAC CTG
CAG AAG AA-3';
```

553-629 (in M. genavense 16S rRNA sequence; corresponding approximately to positions 600-650 in E. coli 16S rRNA sequence):

```
5'-TTT GTC GCG TTG TTC GTG AAA ACC GGG GGC TTA ACC CTC GGC
GTG CGG GCG ATA CGG GCA GAC TGG AGT ACT GCA GG-3';
```

962-1006 (in M. genavense 16S rRNA sequence; corresponding approximately to positions 1000-1040 in E. coli 16S rRNA sequence):

```
5'-ATG CAC AGG ACG CCG GCA GAG ATG TCG GTT CCC TTG TGG CCT
GTG-3';
```

1079-1170 (in M. genavense 16S rRNA sequence; corresponding approximately to positions 1115-1218 in E. coli 16S rRNA sequence):

```
5'-TTG TCT CAT GTT GCC AGC GGG TAA TGC CGG GGA CTC GTG AGA
GAC TGC CGG GGT CAA CTC GGA GGA AGG TGA GGA TGA CGT CAA
GTC ATC AT-3'.
```

Most preferred are the following specific nucleic acid oligonucleotide probes and primers which may be obtained by comparison of the variable regions of the 16S rRNA gene sequence of M. genavense, described above, with 16S rRNA sequences of other mycobacteria described to date, as well as other

EP 0 529 985 A1

eubacterials (the numbers in parentheses are the target regions in the 16S rRNA gene of M. genavense in respect to the aligned 16S rRNA sequence of E. coli):

Probe/primer 420: 5'-CAC GGA ACG CAT GTT TTG TGG-3' (188-211);
Probe/primer reverse 420: 5'-CCA CAA AAC ATG CGT TCC GTG-3' (211-188);
Probe/primer 421: 5'-ATA TGA CCA CGG AAC GCA TGT-3' (180-203);
Probe/primer reverse 421: 5'-ACA TGC GTT CCG TGG TCA TAT-3' (203-180);
Probe/primer 422: 5'-AAC GCA TGT TTT GTG GTG GAA-3' (193-216);
Probe/primer reverse 422: 5'-TTC CAC CAC AAA ACA TGC GTT-3' (216-193);
Probe/primer 423: 5'-ACC GGA TAT GAC CAC GGA AC-3'; (175-195);
Probe/primer reverse 423: 5'-GTT CCG TGG TCA TAT CCG GT-3' (195-175);
Probe/primer 424: 5'-GTT TTG TGG TGG AAA GCT TT-3' (200-224);
Probe/primer reverse 424: 5'-AAA GCT TTC CAC CAC AAA AC-3' (224-200); and
Probe/primer 425: 5'-AGG ATG ACG TCA AGT CAT CAT-3' (1196-1218);
Probe/primer reverse 425: 5'-ATG ATG ACT TGA CGT CAT CCT-3' (1218-1196).

It should be noted that any part of the above sequences of at least about 10 nucleotide bases in length and capable of hybridizing to or amplifying (sense or anti-sense) the 16S rRNA (rDNA) sequence of M. genavense, is embraced by the present invention. For instance, a sequence of nucleic acid which begins in the middle of one of the above described sequences and extends further than the end of that sequence, and which detects M. genavense without any appreciable cross-hybridization with other species, is suitable for use in the invention. Overlapping sequences, combinations of smaller subsequences found within the sequences described above, etc. are also included. Additional sequences that do not interfere with the hybridization abilities of the probe are also included, such as primer sequences, cloning site sequences, and any other type of flanking sequence.

The use of specific nucleic acid primers derived from the 16S rRNA sequence of M. genavense to amplify a portion of this gene, optionally followed by hybridization with an internal nucleic acid probe, and useful for the identification of M. genavense, are also embraced by the present invention, as indicated in Example 5, hereinafter.

The nucleotide sequences contained in the probes of the invention may be characterized as DNA or RNA. Also, the target nucleic acid of M. genavense detected by said probes may be DNA or RNA. Thus RNA probes (either sense or antisense) comprise a labeled nucleic acid complementary to and capable of hybridising to an rRNA fragment comprising all or a part of at least one nucleotide sequence as follows:

5'-ACC GGA UAU GAC CAC GGA ACG CAU GUU UUG UGG UGG AAA GCU UUU GCG G-3';

5'-AAA CCU CUU UCA GCA GGG ACG AAG CGC AAG UGA CGG UAC CUG CAG AAG AA-3';

5'-UUU GUC GCG UUG UUC GUG AAA ACC GGG GGC UUA ACC CUC GGC

EP 0 529 985 A1

GUG CGG GCG AUA CGG GCA GAC UGG AGU ACU GCA GG-3';

5'-AUG CAC AGG ACG CCG GCA GAG AUG UCG GUU CCC UUG UGG CCU
GUG-3';

5'-UUG UCU CAU GUU GCC AGC GGG UAA UGC CGG GGA CUC GUG AGA
GAC UGC CGG GGU CAA CUC GGA GGA AGG UGA GGA UGA CGU CAA
GUC AUC AU-3';

It is preferred that the complementary nucleic acid of M. genavense detected is ribosomal DNA.

RNA or DNA probes may be synthesized by any suitable chemical or biological method. RNA probes may be made by transcription of the corresponding DNA sequences and DNA probes may be synthesized by automated phosphoramide chemistry using cyanoethyl phosphoramides (Beaucage and Carruthers, Tetrahed. Lett. 24:245, 1981).

Labeling of the probes may be performed by any procedure conventional in the art. Generally, we prefer that a labeled substance, such as an enzyme, radioisotope, fluorescent agent or luminescent agent for example, is combined with the DNA or RNA probe. Any form of suitable linkage may be used in this combination and the link may be direct or indirect, for example via a linker such an antibody or avidin.

The nucleic acid probes of the invention may also comprise a derivative, subset, subclone or mutation of at least one of the discrete nucleotide sequences as described above.

The present invention further provides the use of a probe specific for the 16S rRNA sequence of M. genavense in the detection of M. genavense infection in a human or animal subject.

The probes may be used on DNA or RNA from clinical samples, i.e. blood, biopsy or material derived from the patients, with the use of PCR amplification of the 16S rRNA gene sequence, partially or totally, using universal (eubacterial), mycobacterial-specific or M. genavense-specific primers. Subsequent hybridization of PCR products by a specific oligonucleotide probe in solid support and suitable for automation are embraced by the present invention. Procedures such as reverse transcription of rRNA using a short (about 12-mer) oligonucleotide for conversion into cDNA before the PCR assay combined with hybridization which allows the detection of as few 3 mycobacteria or 0.1 fg of nucleic acids, as already described for M. tuberculosis (J. Clin. Microbiol. 28:1751-1759, 1990) may also be adopted or adapted.

Other assays to detect 16S rRNA or rDNA of M. genavense specific sequences may be used. Such techniques include the detection of specifically amplified DNA sequences by immuno assay (Clin. Chem. 37:422-429, 1991; J. Clin. Microbiol. 28:1968-1973 1990), sandwich (dual probe) hybridization (J. Clin. Microbiol. 29:638-641 1991), the hybridisation protection assay (AIDS Res. Hum. Retroviruses 6:1323-1329, 1990). Amplification with PCR may be combined with nonisotopic detection, using the reverse dot blot method, as described in PNAS, USA, 86:6230-6234, 1989, and improvement of specificity may be made by detection of amplified sequences internal to the primers. Detection may also be performed by use of microtiter plates (Anal. Biochem. 177:27-32, 1989) which uses microtiter plates to which captured DNA has been covalently bonded.

Numerous methods also exist for amplification of nucleic acid. A particular example of an alternative method to the PCR method is the ligase chain reaction (LCR) method, which depends on the ligation of oligonucleotides rather than the synthesis of new DNA (ASM News 57:183-186, 1991 and Genomics 4:560-569, 1989). RNA-Qβ probe amplification is an amplification method which relies on the replicative functions of nucleic acids and depends on the incorporation of sequences within the probe (RNA or DNA) that are initiation sites for replicative or transcriptional enzymes (ASM News and Genomics, as above).

The approach and methods outlined in the present invention should provide a rapid and reliable (specific and sensitive) identification of M. genavense from clinical samples or clinical-derived samples. Identification can be made within, for example, 1-2 days.

Knowledge of the 16S rRNA sequence of M. genavense and the production of specific probes/primers, as described in the present invention, allows the clinical diagnosis of M. genavense infection from human or animal sources. Diagnosis involves removal of DNA from samples such as blood, urine, biopsy or any other fluid or tissue, amplification with primers and hybridization with an appropriate probe, for example as hereinafter described.

7

Also embraced by the present invention are assays employing a probe specific for the 16S rRNA sequence of M. genavense. The present invention further provides kits comprising at least one probe specific for the 16S rRNA sequence of M. genavense and useful in the detection of M. genavense infection in a human or animal subject.

EXAMPLE 1

Amplification and sequencing of the 16S rRNA gene of M. genavense from clinical samples and liquid Middlebrook 13A blood cultures

18 patients with advanced AIDS were studied, most of whom suffered from a chronic illness characterized by fever, diarrhoea, and massive loss of weight. All 18 patients were HIV-seropositive and immunosuppressed with extremely low CD4 T-lymphocyte cell counts.

Biopsy and autopsy samples revealed abundant acid-fast micro-organisms in the intestine, liver, spleen, lymph nodes and many other tissues. These micro-organisms, however, did not grow in solid culture media.

Cultures of blood or of biopsies in liquid Middlebrook 13A medium (Bactec System, Becton Dickinson, Towson, MD, USA) showed limited growth in 16 cases. Subculture in solid media was unsuccessful. In addition to blood-culture bottles, acid-fast microorganisms were seen in bone marrow (in all four patients who had this examination), intestinal biopsies (4/4), lymph nodes (6/6), liver (2/2) and multiple other tissues at autopsy (2/2), but again, culture-based attempts at identification failed.

DNA was extracted, using techniques commonly available in the art, from liquid Middlebrook 13A blood cultures of 11 patients, and from liver, spleen, lymph nodes, and/or polymorphonuclear leukocytes (PMN's, buffy coats) of 7 patients.

Oligonucleotides were synthesized on a Gene Assembler Plus synthesizer (Pharmacia, Freiburg, Germany) and purified by shadow-casting polyacrylamide gels (Ausubel F.M. et al. (eds.), Current Protocols in Molecular Biology, John Wiley & Sons Inc, New York, 1987).

The following 20-mer primers were used (numbers in brackets are target sequences corresponding to positions in respect of the E. coli 16S rRNA):

Specific for mycobacteria:

Primer 248: 5'-GTG TGG GTT TCC TTC CTT GG-3' (830-850)

Primer reverse 264: 5'-TGC ACA CAG GCC ACA AGG GA-3' (1046-1027)

Universal for eubacteria:

Primer 246: 5'-AGA GTT TGA TCC TGG CTC AG-3' (8-28)

Primer reverse 261: 5'-AAG GAG GTG ATC CAG CCG CA-3' (1542-1523)

Primer reverse 240: 5'-CCG TCA ATT CCT TTG AGT TT-3' (928-908)

Primers 240, 246, 261 and 264 have been described (Nucleic Acids Res. 17:7843-7853, 1989; J. Clin. Microbiol. 28:1751-1759, 1990). Primer 248 was designed on the basis of published mycobacterial 16S rRNA sequences (Int. J. Syst. Bacteriol. 40:323-330; J. Bacteriol. 172:116-124, 1990).

The polymerase chain reaction (PCR) was performed in a total volume of 50 $\mu$l with 1.25 U of Taq polymerase (Perkin-Elmer Cetus, Norwalk, Conn., USA), 50 mM KCl, 10 mM Tris hydrochloride (pH 8.3), 1.5 mM MgCl$_2$, 0.01% w/v gelatin, 50 pmol of each of two primers and 200 $\mu$M of each of dATP, dCTP, dGTP and dTTP. The 45 $\mu$l mixture was covered by 50 $\mu$l of light mineral oil (Perkin-Elmer Cetus, Norwalk, Conn., USA). DNA was always added last in a volume of 5 $\mu$l while the reaction mixture was kept at 70°C. The thermal profile involved 39 cycles of denaturation at 94°C for 1 min, primer annealing at 64°C for 2 min and extension at 72°C for 6 min. Negative controls were interspersed with clinical samples during every amplification and consisted of reaction mixtures with DNA extracted from blank samples (to control for contamination during DNA extraction) and of PCR reaction mixtures without added DNA. Additional controls contained DNA extracted from uninfected human liver and lymph nodes. The adequacy of DNA extraction and amplification from human tissue was ascertained by amplifying the $\beta$-globin gene. Aliquots of amplified samples (10 $\mu$l) were electrophoresed through 0.8% agarose gels, and the DNA was visualized by UV fluorescence after staining with ethidium bromide.

After 39 cycles of PCR, using 2 different primer pairs (primer 264 plus primer 246; or primer 248 plus primer 261), amplified products of the appropriate size were detected with liver, spleen or PMNs of Patient 1. Urine or centrifuged serum were amplification-negative; nor did they reveal acid-fast organisms on microscopic examination. DNA from tissue (spleen, lymph node, liver) from Patient 2 was also extracted and amplified using the same combination of primers. An agarose-gel electrophoresis of PCR products from samples of patients one and two is shown in Figure 1.

Figure 1:- Agarose-gel electrophoresis of amplified DNA. DNA was amplified using primers specific for mycobacteria in combination with universal primers. Lanes 1-8 and 15-20 show amplification of the 5'-end, and lanes 9 to 14 amplification of the 3' end of the 16S rRNA gene. Sources of amplified DNAs were urine (lane 1), supernatant serum after centrifugation (lane 2), polymorphonuclear leucocytes (lanes 3 and 9), spleen (lanes 4 and 10), liver (lanes 5 and 11) of patient 1; liver (lane 17), lymph node (lane 18), and spleen (lane 19) of patient 2. Lanes 8, 13 and 15 are positive controls and contain DNA isolated from cultures of M. tuberculosis. The remaining six lanes were negative controls: in 6, 12 and 16, extractions from blank samples were added to PCR reagents; in 7, 14 and 20, PCR reagents were processed without additions.

The amplified PCR products were sequenced, revealing an identical nucleotide composition for DNA extracted from liver, spleen and PMNs of Patient 1, which was in turn identical to that of DNA extracted from spleen, lymph node and liver from the unrelated Patient 2. Sequencing of amplified gene fragments of patient 1 and 2 gave a continuous stretch of 1449 nucleotide positions (bases) covering 95% of the 16S rRNA gene (EMBL, accession No. X60070). The sequence is as follows:

```
5'-GTT TGA TCC TGG CTC AGG ACG AAC GCT GGC GGC GTG CTT AAC

ACA TGC AAG TCG AAC GGA AAG GCC TCT TCG GAG GTA CTC GAG

TGG CGA ACG GGT GAG TAA CAC GTG GGT AAT CTG CCC TGC ACT

TCG GGA TAA GCC TGG GAA ACT GGG TCT AAT ACC GGA TAT GAC

CAC GGA ACG CAT GTT TTG TGG TGG AAA GCT TTT GCG GTG TGG

GAT GGG CCC GCG GCC TAT CAG CTT GTT GGT GGG GTG ACG GCC

TAC CAA GGC GAC GAC GGG TAG CCG GCC TGA GAG GGT GTC CGG

CCA CAC TGG GAC TGA GAT ACG GCC CAG ACT NCT ACG GGA GGC

AGC AGT GGG GAA TAT TGC ACA ATG GGC GCA AGC CTG ATG CAG

CGA CGC CGC GTG GGG GAT GAC GGC CTT CGG GTT GTA AAC CTC

TTT CAG CAG GGA CGA AGC GCA AGT GAC GGT ACC TGC AGA AGA

AGC ACC GGC CAA CTA CGT GCC AGC AGC CGC GGT AAT ACG TAG

GGT GCG AGC GTT GTC NGG AAT TAC TGG GCG TAA AGA GCT CGT

AGG TGG TTT GTC GCG TTG TTC GTG AAA ACC GGG GGC TTA ACC

CTC GGC GTG CGG GCG ATA CGG GCA GAC TGG AGT ACT GCA GGG

GAG ACT GGA ATT CCT GGT GTA GCG GTG AAA TGC GCA GAT ATC

AGG AGG AAC ACC GGT GGC GAA GGC GGG TCT CTG GGC AGT AAC

TGA CGC TGA GGA GCG AAA GCG TGG GGA GCG AAC AGG ATT AGA

TAC CCT GGT AGT CCA CGC NGT AAA CGG TGG GTA CTA GGT GTG

GGT TTC CTT CCT TGG GAT CCG TGC CGT AGC TAA CGC ATT AAG

TAC CCC GCC TGG GGA GTA CGG CCG CAA GGC TAA AAC TCA AAG
```

```
GAA TTG ACG GGG GNC CGC ACA AGC GGC GGA GCA TGT GGA TTA
ATT CGA TGC GAC GCG AAG AAC CTT ACC TGG GTT TGA CAT GCA
CAG GAC GCC GGC AGA GAT GTC GGT TCC CTT GTG GCC TGT GTG
CAG GTG GTG CAT GGC TGT CGT CAG CTC GTG TCG TGA GAT GTT
GGG TTA AGT CCC GCA ACG AGC GCA ACC CTT GTC TCA TGT TGC
CAG CGG GTA ATG CCG GGG ACT CGT GAG AGA CTG CCG GGG TCA
ACT CGG AGG AAG GTG AGG ATG ACG TCA AGT CAT CAT GCC CCT
TAT GTC CAG GGC TTC ACA CAT GCT ACA ATG GCC AGT ACA AAG
GGC TGC GAT GCC GTA AGG TTA AGC GAA TCC TTT TAA AGC CGG
TCT CAG TTC GGA TCG GGG TCT GCA ACT CGA CCC CGT GAA GTC
GGA GTC GCT AGT AAT CGC AGA TCA GCA ACG CTG CGG TGA ATA
CGT TCC CGG GCC TTG TAC ACA CCG CCC GTC ACG TCA TGA AAG
TCG GTA ACA CCC GAA GCC AGT GGC CTA ACC TTT TGG AGG GAG
CTG TCG AAG GTG GGA TCG GCG-3'.
```

[N stands for an undetermined base, i.e. either G (guanine), C (cytosine), A (adenine) or T (thymine).]

This sequence was different from any known sequence in the EMBL data base, and showed a high degree of homology to 16S rRNA sequences of other mycobacteria (93.2 to 99.2%) (Table 1).

TABLE 1

| Homology of 16S rRNA genes between M. genavense and other mycobacteria | |
|---|---|
| Species | Percent homology |
| M. simiae | 99.2% |
| M. fortuitum | 96.3% |
| M. smegmatis | 96.1% |
| M. flavescens | 96.1% |
| M. gordonae | 96.1% |
| M. scrofulaceum | 96.0% |
| M. szulgai | 96.0% |
| M. malmoense | 95.8% |
| M. paratuberculosis | 95.8% |
| M. avium | 95.8% |
| M. terrae | 95.6% |
| M. gastri | 95.6% |
| M. marinum | 95.5% |
| M. intracellulare | 95.5% |
| M. tuberculosis (bovis) | 95.5% |
| M. leprae | 94.8% |
| M. nonchromogenicum | 94.6% |
| M. xenopi | 93.2% |

Inspection of the sequence revealed the presence of regions which are characteristic for the genus Mycobacterium as well as sequences which are unique. The sequences therefore suggest that the microorganism is a new member of the genus Mycobacterium for which the name Mycobacterium genavense sp. nov. (from the latin name of Geneva, Genava) is proposed, its closest relative being M. simiae.

During our studies the use of a combination of universal primers (e.g. 246 and 240) for PCR resulted in an amplification signal even without adding DNA. This result possibly reflects the contamination of commercially available Taq polymerase with bacterial DNA as previously reported (Clin. Chem. 36:1258-1259, 1990). After irradiation of the reaction mixtures with UV light (before adding the sample) in order to reduce the quantity of contaminating nucleic acids, tissues of patient 1 yielded an unambiguous sequence of M. genavense even when using universal primers, while the controls (no DNA added to the reactions mixtures) became negative. Thus, using specific mycobacterial primers does not seem to have biased the results against discovering a non-mycobacterial agent.

The sequence of M. genavense shows patterns with unique signature sequences particularly within the following groups (variable regions, in respect to positions in the E. coli 16S rRNA sequence):

positions 157-205 (in M. genavense 16S rRNA sequence; corresponding approximately to positions 175-232 in E. coli 16S rRNA sequence):

```
5'-ACC GGA TAT GAC CAC GGA ACG CAT GTT TTG TGG TGG AAA GCT
TTT GCG G-3';
```

positions 414-463 (in M. genavense 16S rRNA sequence; corresponding approximately to positions 440-500 in E. coli 16S rRNA sequence):

```
5'-AAA CCT CTT TCA GCA GGG ACG AAG CGC AAG TGA CGG TAC CTG
CAG AAG AA-3';
```

positions 553-629 (in M. genavense 16S rRNA sequence; corresponding approximately to positions 600-650 in E. coli 16S rRNA sequence):

```
5'-TTT GTC GCG TTG TTC GTG AAA ACC GGG GGC TTA ACC CTC GGC
GTG CGG GCG ATA CGG GCA GAC TGG AGT ACT GCA GG-3';
```

positions 962-1006 (in M. genavense 16S rRNA sequence; corresponding approximately to positions 1000-1040 in E. coli 16S rRNA sequence):

```
5'-ATG CAC AGG ACG CCG GCA GAG ATG TCG GTT CCC TTG TGG CCT
GTG-3';
```

and positions 1079-1170 (in M. genavense 16S rRNA sequence; corresponding approximately to E. coli position 1115-1218):

```
5'-TTG TCT CAT GTT GCC AGC GGG TAA TGC CGG GGA CTC GTG AGA
GAC TGC CGG GGT CAA CTC GGA GGA AGG TGA GGA TGA CGT CAA
GTC ATC AT-3'.
```

The alignment with published mycobacterial 16S rRNA (rDNA) sequences (Int. J. Syst. Bacteriol. 40:323-330; FEMS Microbiology Lett. 64:231-237, 1991; J. Bacteriol. 172:116-124, 1990 and those found in the EMBL data base) was done by using the alignment algorithm of Krüger and Osterburg (Comput. Methods Programs Biomed. 16:61-70, 1983). This algorithm was manually adjusted to account for common secondary structures.

These findings were applied to patients 3-18. DNA was amplified from the blood culture bottles (11 patients), lymph nodes (4 patients), and bone marrow (1 patient). Sequences for two of the variable regions

# EP 0 529 985 A1

(J. Gen. Microbiol. 136:1915-1920, 1990) were determined: positions 120-280 and 400-450. Analysis showed that the characteristic signature sequences of M. genavense were present as shown in Table 2.

TABLE 2:- Alignment of mycobacterial 16S rRNA gene sequences.

```
TAA TAC CGG ATA GG-ACCA CGG GAT GCA TG--TCT-TGT GGT GGA AAG C   M. tuberculosis
... ... ... ... T.-.... ... A.C ... ..---.T.-... ... ... ... .   M. genavense
... ... ... ... ..-.... .TT .GC ... ...--C...-... ... ... ... .   M. simiae
... ... ..A ... T.-.... ..C .C. T.. ..G-..G.--... ... ... ... .   M. fortuitum
... ... ..A ... TT-C..T NT. .TC ...   .GCCTGG.A- ..- ... ... .   M. flavescens
... ... ..A ... CACC.TG .T. .TC ... ..GCCTGG.A- ..- ... ... .   M. smegmatis
... ... ..A ... ..-...G .AT .C. ... ...--GTG-... ... ... ... .   M. nonchromogenicum
... ... ... ... ..-.... T.. ... ... ...-T...--... ... ... ... .   M. terrae
... ... ... ... ..-.... TTC TGC ... ..---.GG-G.. ... ... ... .   M. xenopi
... ... ... ... ..-..TT .AA .GC ... ..--...-... ... ... ... .   M. leprae
... ... ... ... ..-.... .TT .GC ... ...--C...-... ... ... ... .   M. kansasii
... ... ... ... ..-.... .TT .GC ... ...--C..-... ... ... ... .   M. gastri
... ... ... ... ..-...T .AA .GC ... ...--C..T... ... ... ... .   M. haemophilum
... ... ..A ... ..-.... .A. ..C A.. ..---.C-... ... ... ... .   M. gordonae
... ... ... ... ..-...T .AA ..C ... ..-...-.C. ... ... ... .   M. avium
... ... ... ... ..-...T .AA ..C ... ..-...-.C. ... ... ... .   M. paratuberculosis
... ... ... ... ..-...T TTA .GC ... ..-...-.TA ... ... ... .   M. intracellulare
... ... ..A ... ..-...C ..A .GC ... ...--C..-..G ... ... ... .   M. malmoense
... ... ... ... ..-...C ..A .GC ... ...--C..-..G ... ... ... .   M. szulgai
... ... ... ... ..-.... ... ... T.. ..--..C-... ... ... ... .   M. marinum
... ... ..A ... T.-.T.. T.C TC. T.. ...--GGG-... ... ... ... .   M. sphagni
... ... ... ... ..-..T. ... AC. ... ..G-...-... ... ... ... .   M. cookii
... ... ... ... T.-.... T.. AC. T.. C.G-.T.--... ... ... ... .   M. kommossense
... ... ... ... ..-.... ..C .C. T.. ..G-..G.--... ... ... ... .   M. farcinica
... ... ... ... CACC.TT .T. .T. ... ..G--...-G.G A.G ... ... .   M. phlei
... ... ..A ... T.-...G ..C AC. T.C ..G-..G.--... ... ... ... .   M. chelonae
... ... ... ... ..-.... .TT .GC ... ...--C..-... ... ... ... .   M. scrofulaceum
... ... ... ... ..-.... .AT .TC ... ...--GTG-... ... ... ... .   M. triviale
... ... ..A ... CACC.TG .T. .TC ... ..GCCTG--... ..G ... ... .   M. thermoresistible
... ... ... ... ..-.T.. T.C .CC T.. ...--GTG-... ... ... ... .   M. fallax
... ... ..A ... ..-..T. ..C ... ... ..---.G-... ... ... ... .   M. aurum
... ... ... ... ..-.... ..C .C. T.. ...--GTG-... ... ... ... .   M. senegalense
... ... ... ... ..-.... ... ... ... ..---.C-... ... ... ... .   M. asiaticum
```

Table 2:- Alignment of mycobacterial 16S rRNA sequences demonstrating signature sequences for M. genavense in the V2 region (Dams E. et al., Nucleic Acids Research 16 (supplement): s87-s175, 1988). The first nucleotide in the figure corresponds to position 171 of the E. coli 16S rRNA gene. M. tuberculosis was used as the source of reference sequence. Only nucleotides different from M. tuberculosis are shown; dashes indicate deletions.

12

Infections with M. genavense may be more frequent than the cases described here. Several unlikely events had to occur for us to identify an isolate, namely microscopical detection of acid-fast rods in normally sterile tissue; some growth in blood cultures; and the availability, months later and after efforts at identification had failed, of the original blood culture. Furthermore, this organism is likely to be found more frequently now that zidovudine and Pneumocystis carinii pneumonia prophylaxis have changed the course of HIV infection and AIDS such that more patients with very low CD4 T-cell counts survive without pneumocystis pneumonia. M. genavense should be considered seriously in the differential diagnosis of patients with CD4 T-cell counts below 100/$\mu$l and diarrhoea, weight loss, and fever.

The natural reservoir and the epidemiology of M. genavense remain to be established. The striking gastrointestinal symptoms and signs suggest the gut as the reservoir from which M. genavense invades others tissues. The use of M. genavense specific sequences in combination with PCR driven by mycobacterial primers, followed by hybridisation with M.genavense 16S rRNA-derived probes, may permit rapid, sensitive and specific detection of M. genavense, and an assessment of its prevalence in symptomatic and symptomless HIV infection and in HIV-seronegative patients with depressed or normal immunity.

EXAMPLE 2

Specificity of amplification reaction products: slot blot hybridization with genus and species-specific mycobacterial probes

The specificity of the 16S rRNA sequence amplification was further studied by slot blot hybridization with selected oligonucleotide probes by comparing the hybridization of specific probes for the genus Mycobacterium, for M. tuberculosis and 3 probes for M. genavense with amplified products from several mycobacterial species including 7 M. genavense samples from 7 different patients. Small aliquots of amplified samples (10 $\mu$l) were electrophoresed through 0.8% agarose gels, and the DNA was visualized in the gels after staining with ethidium bromide.

For slot blot analysis, 20 $\mu$l (1 $\mu$g of DNA) of amplified samples or nucleic acids from each organism tested were denatured by adding 100 $\mu$l of 0.5 M NaOH and 25 mM EDTA, incubated for 35 minutes at room temperature, and neutralized with 120 $\mu$l of ammonium acetate. The samples were loaded into wells of a manifold (Minifold II, Schleicher & Schüll, Dassel, Germany) fitted with a nitrocellulose membrane (BA 85; Schleicher & Schüll) previously wetted in 10X SSC (1X SSC = 0.15 M NaCl + 0.015 M sodium citrate, pH 7). Each well was then rinsed with 0.4 ml of 10X SSC, and the membranes were heated for 2 h at 80°C for drying the samples and to fix the nucleic acids on the membranes. Hybridizations was performed by standard techniques. The membranes were rinsed in 6X SSC and prehybridized overnight in a hybridization mixture containing 6X SSC-5X Denhardt solution (1X Denhardt = 0.02% Ficoll-0.02% polyvinylpirrolidone-0.02% bovine serum albumin)-0.1% sodium dodecyl sulfate (lauryl sulfate)-100 $\mu$g/ml of tRNA (Boehringer, Mannheim, Germany) for 1 to 2 hours at 50°C. After prehybridization, the hybridization mixture was discarded and replaced by fresh hybridization mixture (the same as above) and hybridization was performed by adding into this mixture 2 x $10^6$ to 1 x $10^7$ cpm/ml of 5'-end labeled oligonucleotide probe overnight. The membranes were then washed three times in 6X SSC (20 min each) and three times (20 min each) at the appropriate temperature given by the Tm (melting temperature) in degrees Celsius of the oligonucleotide. The blots were exposed to XAR-5 film (Eastman Kodak Co, Rochester, NY) in cassettes containing an intensifying screen for various lengths of time at -70°C.

Oligonucleotides were synthesized on a Gene Assembler Plus (Pharmacia) and purified with a shadow-casting polyacrylamide gel.

The labeling of the oligonucleotide probes was performed using standard techniques. Probes were labeled in 15 to 20 $\mu$l of 50 mM Tris (pH 7.6)-10 mM MgCl$_2$-5 mM dithiothreitol-0.1 mM spermidine-0.1 mM EDTA with 8 pmol of synthetic oligonucleotide. 100 $\mu$Ci of [$\gamma$-$^{32}$P]ATP (specific activity, higher than 5,000 Ci/mmol, Amersham), and 10 U of T4 polynucleotide kinase (New England Biolabs) by incubation for 1 h at 37°C. The activity of the kinase enzyme was then inactivated by incubation for 10 min at 75°C; the oligonucleotides were extracted with phenol, and the labeled oligonucleotide probes were purified and separated from residual [$\gamma$-$^{32}$P]ATP with push columns (Stratagene, Heidelberg, Germany). The specific activity of the labeled oligonucleotide probes were 2 x $10^8$ to 5 x $10^8$ cpm/$\mu$g.

The PCR reactions were performed using oligonucleotide primers:

246 (5'-AGA GTT TGA TCC TGG CTC AG-3'); and reverse 264 (5'-TGC ACA CAG GCC ACA AGG GA-3').

The presence of human DNA in mycobacterial samples was also tested and it did not interfere with the detection of the mycobacteria.

The following probes were used:

Probe reverse 247 (mycobacteria): 5'-TTT CAC GAA CAA CGC GAG AA-3'

Probe reverse Tub (M. tuberculosis, positions corresponding to E. coli 16S rRNA: 212-192): 5'-ACC ACA AGA CAT GCA TCC CG-3'

Probe/primer reverse 420 (M. genavense): 5'-CCA CAA AAC ATG CGT TCC GTG-3'

Probe/primer reverse 421 (M. genavense): 5'-ACA TGC GTT CCG TGG TCA TAT-3'

Probe/primer reverse 422 (M. genavense): 5'-TTC CAC CAC AAA ACA TGC GTT-3'

TABLE 3:- Slot blot hybridization with oligonucleotide probes for mycobacterial, <u>M. tuberculosis</u> and <u>M. genavense</u> 16S rRNA genes

| Microorganism | Probe reverse 247 | Probe reverse Tub | Probe reverse 420 | Probe reverse 421 | Probe reverse 422 |
|---|---|---|---|---|---|
| <u>M. tuberculosis</u> | + | + | - | - | - |
| <u>M. bovis</u> | + | + | - | - | - |
| <u>M. bovis</u> BCG | + | + | - | - | - |
| <u>M. tuberculosis</u> H37 | + | + | - | - | - |
| <u>M. avium</u> Chester DSM 43216 | + | - | - | - | - |
| <u>M. africanum</u> | + | + | - | - | - |
| <u>M. chelonae</u> ATCC 14472 | + | - | - | - | - |
| <u>M. cookii</u> | + | - | - | - | - |
| <u>M. farcinica</u> DSM 43294 | + | - | - | - | - |
| <u>M. fortuitum</u> ATCC 6841T | + | - | - | - | - |
| <u>M. gastri</u> ATCC 15754T | + | - | - | - | - |

| | | | | | |
|---|---|---|---|---|---|
| M. gordonae ATCC 14470T | + | - | - | - | - |
| M. intracellulare ATCC 15985 | + | - | - | - | - |
| M. kansasii DSM 43224 | + | - | - | - | - |
| M. komossense ATCC 33013 | + | - | - | - | - |
| M. marinum | + | - | - | - | - |
| M. malmoense ATCC 29571T | + | - | - | - | - |
| M. paratuberculosis ATCC 19698 | + | - | - | - | - |
| M. phlei ATCC 35784 | + | - | - | - | - |
| M. scrofulaceum ATCC 19981T | + | - | - | - | - |
| M. simiae ATCC 25275T | + | - | - | - | - |
| M. sphagni ATCC 33027T | + | - | - | - | - |
| M. smegmatis ATCC 14468 | + | - | - | - | - |
| M. szulgai ATCC 25799T | + | - | - | - | - |
| M. terrae ATCC 15755T | + | - | - | - | - |
| M. genavense Patient 1 | + | - | + | + | + |
| M. genavense Patient 2 | + | - | + | + | + |
| M. genavense Patient 8 | + | - | + | + | + |
| M. genavense Patient 11 | + | - | + | + | + |
| M. genavense Patient 13 | + | - | + | + | + |
| M. genavense Patient 14 | + | - | + | + | + |
| M. genavense Patient 16 | + | - | + | + | + |
| Nocardia asteroides | - | - | - | - | - |
| Actinomyces israelii | - | - | - | - | - |
| Propionibacterium acnes | - | - | - | - | - |

ATCC, American Type Culture Collection, Rockville, USA;
DSM, Deutsche Stammsammlung für Mikroorganismen,
Braunschweig, Germany; T, Type strain.
+, strong positive hybridization; -, negative
hybridization.

Probe reverse 247: hybridization at 52°C, washings at 55°C.
Probe reverse Tub: Hybridization at 52°C, washings at
61°C.

Probe reverse 420: hybridization at 52°C, washings at 61°C.
Probe reverse 421: hybridization at 52°C, washings at 59°C.
Probe reverse 422: hybridization at 52°C, washings at 57°C.

Table 3 shows that hybridization of PCR products with probe reverse 247, a probe broadly reactive for mycobacteria, resulted in strong hybridization with all mycobacteria tested including 7 M. genavense samples, whereas samples from Nocardia asteroides, Actinomyces israelii, and Propionibacterium acnes were negative. Use of probe reverse Tub, specific for M. tuberculosis, resulted in strong hybridisation for M. tuberculosis, M. bovis, M. bovis BCG, and M. africanum but not for other mycobacteria or non-mycobacterial samples. This result is not surprising because it is known that these mycobacteria cannot be separated on the basis of the 16S rRNA sequence (Rogall T. et al. J. Gen. Microbiol. 136:1915-1920, 1990). Reactions with probes reverse 420, reverse 421, and reverse 422, specific for M. genavense, gave a strong hydridization signal only with the M. genavense samples and not with the other mycobacterial and non-mycobacterial samples, confirming the specificity of the probes designed on basis of the 16S rRNA sequence.

EXAMPLE 3

Sensitivity of amplification reactions and slot blot hybridization

To determine the sensitivity of the amplification procedure, nucleic acids extracted from sequential dilutions of a suspension containing a known number of M. genavense (bacteria were concentrated by centrifugation and after homogenization of necropsy samples from patient 1) were added to the PCR mixtures performed with primers 246 and reverse 264. Aliquots of the PCR amplification products were electrophoresed through a 0.8% agarose gel, and a band of the expected length was observed in samples containing at least 500 bacteria. The PCR products were blotted into a cellulose membrane using a slot blot apparatus and hybridised with radiolabeled ($^{32}$P) oligonucleotide probe reverse 420 (specific for M. genavense). The results of the hybridization tests using this probe showed that starting samples containing 50 bacteria gave a strong hybridization pattern whereas samples containing as few as 5 mycobacteria gave a positive, but less marked, hybridization pattern. This demonstrates that by combining the PCR amplification procedure with hybridization of the PCR products with a specific-probe for M. genavense sensitivity is increased 10- to 100-fold.

EXAMPLE 4

Detection and identification of M. genavense infection by reverse transcription of rRNA into cDNA

For increasing sensitivity in detection, the procedures such as reverse transcription of rRNA using a short (about 12-mer) oligonucleotide to convert it into cDNA before the PCR assay combined with hybridization which allows the detection of as few as 3 mycobacteria in samples or when using purified nucleic acids of as few as 0.1 fg of nucleic acids, as previously described for M. tuberculosis (J. Clin. Microbiol. 28:1751-1759, 1990) may be adopted or adapted.

EXAMPLE 5

Highly specific detection and identification of M. genavense

In order to increase the specificity of detection and identification of M. genavense from a sample, the PCR reaction can itself be made more specific. This may be performed by using primers such as the probe/primer 423 (as hereinbefore defined) in conjunction with the probe/primer reverse 425 (as hereinbefore defined). Alternatively a sequence complementary to that of probe/primer 423 can be used in conjunction with the anti-sense sequence of probe/primer 425. Use of either of these combinations directs the synthesis of an M. genavense specific 1,013 base pair gene fragment by PCR. The PCR products may then in turn be probed, for example using a probe internal to the amplified gene fragment, such as the probe/primer 424 (as hereinbefore defined) or the probe/primer reverse 424 (as hereinbefore defined).

**Claims**

1. The 16S rRNA sequence of a new mycobacterial species tentatively called M. genavense sp. nov. and consisting of:

```
5'-GTT TGA TCC TGG CTC AGG ACG AAC GCT GGC GGC GTG CTT

AAC ACA TGC AAG TCG AAC GGA AAG GCC TCT TCG GAG GTA CTC

GAG TGG CGA ACG GGT GAG TAA CAC GTG GGT AAT CTG CCC TGC

ACT TCG GGA TAA GCC TGG GAA ACT GGG TCT AAT ACC GGA TAT

GAC CAC GGA ACG CAT GTT TTG TGG TGG AAA GCT TTT GCG GTG

TGG GAT GGG CCC GCG GCC TAT CAG CTT GTT GGT GGG GTG ACG

GCC TAC CAA GGC GAC GAC GGG TAG CCG GCC TGA GAG GGT GTC

CGG CCA CAC TGG GAC TGA GAT ACG GCC CAG ACT NCT ACG GGA

GGC AGC AGT GGG GAA TAT TGC ACA ATG GGC GCA AGC CTG ATG

CAG CGA CGC CGC GTG GGG GAT GAC GGC CTT CGG GTT GTA AAC

CTC TTT CAG CAG GGA CGA AGC GCA AGT GAC GGT ACC TGC AGA

AGA AGC ACC GGC CAA CTA CGT GCC AGC AGC CGC GGT AAT ACG

TAG GGT GCG AGC GTT GTC NGG AAT TAC TGG GCG TAA AGA GCT

CGT AGG TGG TTT GTC GCG TTG TTC GTG AAA ACC GGG GGC TTA

ACC CTC GGC GTG CGG GCG ATA CGG GCA GAC TGG AGT ACT GCA

GGG GAG ACT GGA ATT CCT GGT GTA GCG GTG AAA TGC GCA GAT

ATC AGG AGG AAC ACC GGT GGC GAA GGC GGG TCT CTG GGC AGT

AAC TGA CGC TGA GGA GCG AAA GCG TGG GGA GCG AAC AGG ATT

AGA TAC CCT GGT AGT CCA CGC NGT AAA CGG TGG GTA CTA GGT

GTG GGT TTC CTT CCT TGG AAT CCG TGC CGT AGC TAA CGC ATT

AAG TAC CCC GCC TGG GGA GTA CGG CCG CAA GGC TAA AAC TCA

AAG GAA TTG ACG GGG GNC CGC ACA AGC GGC GGA GCA TGT GGA

TTA ATT CGA TGC GAC GCG AAG AAC CTT ACC TGG GTT TGA CAT

GCA CAG GAC GCC GGC AGA GAT GTC GGT CCC CTT GTG GCC TGT

GTG CAG GTG GTG CAT GGC TGT CGT CAG CTC GTG TCG TGA GAT

GTT GGG TTA AGT CCC GCA ACG AGC GCA ACC CTT GTC TCA TGT

TGC CAG CGG GTA ATG CCG GGG ACT CGT GAG AGA CTG CCG GGG

TCA ACT CGG AGG AAG GTG AGG ATG ACG TCA AGT CAT CAT GCC

CCT TAT GTC CAG GGC TTC ACA CAT GCT ACA ATG GCC AGT ACA

AAG GGC TGC GAT GCC GTA AGG TTA AGC GAA TCC TTT TAA AGC

CGG TCT CAG TTC GGA TCG GGG TCT GCA ACT CGA CCC CGT GAA

GTC GGA GTC GCT AGT AAT CGC AGA TCA GCA ACG CTG CGG TGA

ATA CGT TCC CGG GCC TTG TAC ACA CCG CCC GTC ACG TCA TGA


AAG TCG GTA ACA CCC GAA GCC AGT GGC CTA ACC TTT TGG AGG

GAG CTG TCG AAG GTG GGA TCG GCG-3'.
```

**2.** A nucleic acid probe capable of hybridizing to, under predetermined stringency conditions, the 16S ribosomal RNA or 16S ribosomal DNA sequence of M. genavense or a sequence complementary thereto.

3. A nucleic acid probe according to claim 2 comprising at least 10 nucleotide positions.

4. A sense or antisense nucleic acid probe or primer selected from the group consisting of the following sequences:

5'-ACC GGA TAT GAC CAC GGA ACG CAT GTT TTG TGG TGG AAA GCT TTT GCG G-3';

5'-AAA CCT CTT TCA GCA GGG ACG AAG CGC AAG TGA CGG TAC CTG CAG AAG AA-3';

5'-TTT GTC GCG TTG TTC GTG AAA ACC GGG GGC TTA ACC CTC GGC GTG CGG GCG ATA CGG GCA GAC TGG AGT ACT GCA GG-3';

5'-ATG CAC AGG ACG CCG GCA GAG ATG TCG GTT CCC TTG TGG CCT GTG-3'; and

5'-TTG TCT CAT GTT GCC AGC GGG TAA TGC CGG GGA CTC GTG AGA GAC TGC CGG GGT CAA CTC GGA GGA AGG TGA GGA TGA CGT CAA GTC ATC AT-3';

or a probe or primer having a nucleotide sequence complementary thereto, each of said probes or primers being capable of hybridizing to, or amplifying, all or part of the 16S ribosomal RNA or rDNA sequence of M. genavense.

5. A sense or antisense nucleic acid probe complementary to, and able to hybridise to, a sequence selected from the group consisting of:

```
5'-ACC GGA UAU GAC CAC GGA ACG CAU GUU UUG UGG UGG AAA GCU
UUU GCG G-3';


5'-AAA CCU CUU UCA GCA GGG ACG AAG CGC AAG UGA CGG UAC CUG
CAG AAG AA-3';


5'-UUU GUC GCG UUG UUC GUG AAA ACC GGG GGC UUA ACC CUC GGC
GUG CGG GCG AUA CGG GCA GAC UGG AGU ACU GCA GG-3';


5'-AUG CAC AGG ACG CCG GCA GAG AUG UCG GUU CCC UUG UGG CCU
GUG-3'; and


5'-UUG UCU CAU GUU GCC AGC GGG UAA UGC CGG GGA CUC GUG AGA
GAC UGC CGG GGU CAA CUC GGA GGA AGG UGA GGA UGA CGU CAA
GUC AUC AU-3';
```

each of said probes being capable of hybridising to M. genavense 16S ribosomal RNA.

**6.** A nucleic acid probe or primer selected from the group consisting of probe/primers 420, reverse 420; 421, reverse 421; 422, reverse 422; 423, reverse 423; 424, reverse 424; 425 or reverse 425, or a probe or primer having a nucleotide sequence complementary thereto, each of said probes being capable of hybridising to, or amplifying, the 16S ribosomal RNA sequence (rDNA) of M. genavense.

**7.** A nucleic acid probe specific for and capable of hybridizing to the nucleotide sequence defined in claim 1.

**8.** Use of a probe as defined in claim 2 in the detection of M. genavense.

**9.** A method for detecting M. genavense in a sample comprising the steps of:
a) contacting the sample with a probe specific for and capable of hybridising with the 16S rRNA or rDNA sequence of M. genavense under conditions that allow the probe to hybridize and complex with said rRNA or rDNA, said probe being unable to hybridize to rRNA or rDNA of non-M. genavense microorganisms;
b) detecting said nucleic acid complexes.

**10.** A method as claimed in claim 9 wherein the probe is a probe as defined in claim 2.

**11.** A method as claimed in claim 9 further comprising the step of amplifying the probe and/or the 16S rRNA or 16S rRNA gene sequences of M. genavense.

FIGURE 1

1  2  3  4  5  6  7  8  9  10  11  12  13  14  15  16  17  18  19  20

— 2.0

— 1.0

— 0.5

European Patent Office

EUROPEAN SEARCH REPORT

Application Number

EP 92 30 7690

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | NUCLEIC ACIDS RESEARCH vol. 17, no. 19, 1989, OXFORD, GB. pages 7843 - 7853 EDWARDS, U. ET AL. 'Isolation and direct complete nucleotide determination of entire genes. Characterization of a gene coding for 16S ribosomal RNA' *Whole article, especially fig 3* | 2-5,8 | C12Q1/68 C07H21/04 C12P19/34 |
| A | FEMS MICROBIOLOGY LETTERS vol. 65, 1989, AMSTERDAM, THE NETHERLANDS pages 171 - 176 BÖTTGER, E. 'Rapid determination of bacterial ribosomal RNA sequences by direct sequencing of enzymatically amplified DNA' * the whole document * | 2-11 | |
| X | WO-A-8 803 957 (GENPROBE INC.) * page 24 - page 57; claims 31-83 * | 2-5,8 | |

TECHNICAL FIELDS SEARCHED (Int. Cl.5)

C12Q

The present search report has been drawn up for all claims

| Place of search | Date of completion of the search | Examiner |
|---|---|---|
| THE HAGUE | 10 NOVEMBER 1992 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)